Europäisches Patentamt

European Patent Office

Office européen des brevets

(11) Publication number: **0 133 044**
**B1**

(12) **EUROPEAN PATENT SPECIFICATION**

(45) Date of publication of patent specification: **07.11.90**

(51) Int. Cl.⁵: **C 12 N 15/00** // C12R1/19

(21) Application number: **84305169.9**

(22) Date of filing: **30.07.84**

(54) Stabilising foreign proteins, and plasmids for use therein.

(30) Priority: **01.08.83 US 518926**

(43) Date of publication of application:
**13.02.85 Bulletin 85/07**

(45) Publication of the grant of the patent:
**07.11.90 Bulletin 90/45**

(84) Designated Contracting States:
**BE CH DE FR GB IT LI NL SE**

(56) References cited:
**EP-A-0 072 925**

**PROCEEDINGS OF THE NATIONAL ACADEMY
OF SCIENCES OF THE UNITED STATES OF
AMERICA, vol. 8o, no.7, April 1983, Baltimore,
USA L.D. SIMON et al. "Stabilization of proteins
by a bacteriophage T4 gene cloned in
Escherichia coli" pages 2059-2062**

**The file contains technical information
submitted after the application was filed and
not included in this specification**

(73) Proprietor: **THE RESEARCH FOUNDATION OF
STATE UNIVERSITY OF NEW YORK
State University Plaza Broadway
Albany New York 12246 (US)**

(72) Inventor: **Shub, David A.
3 Parkwood street
Albany, NY 12203 (US)**
Inventor: **Casna, Nancy J
25 Orlando Avenue
Albany, NY 12203 (US)**

(74) Representative: **Perry, Robert Edward et al
GILL JENNINGS & EVERY 53-64 Chancery Lane
London WC2A 1HN (GB)**

Courier Press, Leamington Spa, England.

## Description

This invention relates to a process for stabilising foreign proteins, and plasmids for use therein.

The use of *Escherichia coli* bacteria as the host in molecular biology is well known. This microbe apparently was the first prokaryote used for the commercial production of insulin via a cloned gene. *E. coli* as a host for a eukaryotic gene encoding a useful polypeptide or protein is not without defects. One defect which received early extensive notoriety is the production of an endotoxin by *E. coli*. The presence of an endotoxin in a preparation of protein product is not desirable. Thus, extensive work has been done to alleviate this problem. Another defect with use of *E. coli* as a host is that the microbe produces proteases which can destroy susceptible protein product produced by the microbe. This destruction of protein product could lead to the wrong conclusion of low levels of expression of some cloned eukaryotic genes. Generally, the proteases act upon incomplete or abnormal proteins produced by the microbe. However, it is known that *E. coli* proteases also act adversely upon useful foreign proteins, for example, human beta-preinterferon and somatostatin.

The publication "Stabilization of proteins by a bacteriophage T4 gene cloned in *Escherichia coli*" by L. D. Simon et al., Proc. Natl. Acad. Sci. USA, Vol. 80, pp. 2059—2062, April 1983, discloses the use of a cloned T4 *pin* gene to stabilize three different kinds of proteins in *E. coli* cells, i.e., (1) incomplete proteins, e.g., puromycyl polypeptides, (2) abnormal but complete proteins, e.g., the λtsO protein, and (3) labile eukaryotic proteins encoded by genes cloned in *E. coli*, e.g., mature human fibroblast interferon. The process described in this publication involves the use of two apparently hybrid plasmids transformed into the *E. coli* host. One plasmid contains the T4 *pin* gene whereas the other contains the eukaryotic gene encoding a desired protein product. Though this process allegedly stabilizes the indicated proteins, it is not a desirable commercial process because of the use of two different plasmids in the *E. coli* host. The compatability of such plasmids may not be as stable as desired in the commercial production of useful proteins by recombinant means. Alteration or loss of compatibility can wreak havoc on a commercial operation.

The process of the subject invention avoids the issue of plasmid incompatibility by use of a doubly chimeric plasmid from which a foreign DNA sequence is recombined into a T-even bacteriophage, which is then used to infect the *E. coli* host. The stabilization aspect of the subject invention is not based on the cloning of the T4 *pin* gene as is the case with the process of Simon *et al.*

Summary of the invention

According to the present invention, in a process for stabilising proteins which can be expressed by *E. coli* via a cloned gene and which may be susceptible to proteases produced within the *E. coli* cell, the process comprises:

(a) constructing a chimeric plasmid comprising a DNA fragment from an *E. coli* plasmid and a T-even bacteriophage which is stably propagated in an *E. coli* bacterium;

(b) inserting foreign DNA, *in vitro*, into the DNA fragment from a T-even bacteriophage of the chimeric plasmid, to give a doubly chimeric plasmid;

(c) recombining, *in vivo*, a T-even bacteriophage and the doubly chimeric plasmid, resulting in the insertion of the foreign DNA into the bacteriophage; and

(d) infecting an *E. coli* bacterium with T-even bacteriophage harbouring the foreign DNA.

The net effect of the novel process is a higher yield of foreign proteins from the *E. coli* cell.

Detailed disclosure of the invention

Abbreviations used herein are as follows: bp, base pairs; CAP, catabolite gene activator protein; DTT, dithiothreitol; EtBr, ethidium bromide; glcHMC, glucosyl-5-hydroxymethyl cytosine; HMC, 5-hydroxymethyl cytosine; HMU, 5-hydroxymethyl uracil; kb, kilobase pairs; Xgal, 3-bromo-4-chloro-5-indolyl-β-galactoside; [], indicates plasmid-carrier state.

Publications cited herein appear in the section "References", after the Examples.

The construction of the chimeric plasmid comprising bacteriophage T-even DNA and a portion of the genome of an *E. coli* plasmid is exemplified herein by use of T4 DNA and the genome of a modified *E. coli* plasmid. Other bacteriophage T-even DNA can be used so long as the DNA can be stably propagated in *E. coli*, i.e., it is not lethal to *E. coli*. This T-even DNA can be an entire gene or a portion of a gene.

Also, though the examples following disclose the insertion of heterologous DNA into the *r*II gene of T4, it should be recognized that such DNA can be cloned into the gene or portions of a gene of any T-even bacteriophage. Further, there are a large number of genes in T4 other than the *r*IIB gene into which the heterologous DNA can be cloned by procedures disclosed herein or others well known in the art.

The subject process is exemplified by use of bacteriophage T4 as the cloning vehicle. It should be understood that other T-even bacteriophages, laboratory or naturally-occurring related strains, can be used to infect the *E. coli* host, so long as they are biologically equivalent to T4, i.e., can serve as a DNA-cloning vehicle in substantially the same manner as disclosed herein for T4.

Any *E. coli* can be used as the host microbe. It may contain an amber suppressor mutation. Such microbes are well known and available to the public.

The Yale University *E. coli* culture collection makes subcultures available to the public upon request. *E.*

coli 1078 used in the disclosure following, as well as E. coli C600 are available from the Yale repository without restriction.

Materials and methods

(a) E. coli strains, phage strains and plasmids

E. coli 1078 (C600 r⁻m⁺ supE44 thr⁻ leu⁻ thi⁻) was used as a recipient for transformation and propagation of plasmid DNA. HR141A (U95 rgl⁻) was used to prepare HMC DNA (Revel and Georgopoulos, 1969). For plating phage: CR63 (sul$_{am}$⁺); CR63 (λ) (sul$_{am}$⁺); BB(su⁰; permissive for rescue of gene 30$_{am}$⁻ by mutations in rII), were used. T4 strain C104B (gene 30$_{am}$⁻) was used for gene 30 rescue experiments.

Plasmid pOP203-1 is a pMB9 derivative that contains a 203-bp HaeIII fragment (containing the UV5 promoter mutation) of the lactose region of E. coli, inserted at the EcoRI site (Backman et al., 1976). Plasmid pBR322 (H23r⁺) contains an 873-bp fragment of the intercistronic region of the rII genes of T4, inserted at the HindIII site of pBR322 (Pribnow et al., 1981).

(b) Enzymes and other reagents

Restriction endonucleases EcoRI, AluI, BglI, HhaI, BamHI (New England Biolabs), HinfI (Bethesda Research labs), and HindII and TaqI (Boehringer Mannheim) were used according to the specifications supplied by the vendor, except that TaqI was used at 4 units per microgram of T4 DNA for 3 hours in order to achieve complete reactions. EcoRI* reactions were carried out at a final concentration of 25 mM Tris · HCl, pH 8.5; 2 mM MgCl$_2$ (Polisky et al., 1975), S1 nuclease (Boehringer Mannheim) reactions were performed with 8 μg of EcoRI-cut pOP203-1 DNA and 80 units of enzyme, in 0.2 ml of 0.2 mM ZnSO$_4$, 50 mM sodium acetate pH 4.5, 100 mM NaCl for 30 min. at 37°C. T4 DNA ligase (BRL) was reacted at a final concentration of 50 mM Tris · HCl pH 7.5, 100 mM MgCl$_2$, 20 mM DTT, 1 mM ATP overnight at 4°C with 1 unit/μg of DNA. Xgal was dissolved in dimethylformamide to 2% and 50 μl was used per agar plate. Klenow fragment of DNA polymerase I (Boehringer Mannheim) was used at 1 unit/μg DNA, to fill in staggered ends at a final concentration of 6.6 mM Tris · HCL pH 7.5, 6.6 mM NaCl, 6.6 mM MgCl$_2$, 6.6 mM DTT and 0.1 mM of each dNTP.

(c) Gel electrophoresis

Analytical agarose gels (1%) were run at a constant voltage of 150 V for an average of 16 h in buffer of final concentration 40 mM Tris · HCl pH 7.9, 20 mM sodium acetate, 1 mM EDTA. The gels were stained with 1 μg/ml EtBr for 15 min and photographed by trans-illumination with longwave UV light.

(d) Preparation of DNA and transformation

HMC T4 DNA was prepared by diluting the progeny of an infection of E. coli HR141A, concentrated and purified by a CsCl step gradient, to an A$_{260}$ of 20. The phage were then extracted twice with an equal volume of phenol saturated with 0.1 M Tris · HCl pH 7.9 and the aqueous phase was dialyzed at 4°C overnight into 10 mM Tris · HCl pH 7.9, 0.1 M NaCl, 0.1 mM EDTA.

Plasmid and M13 RF DNAs were prepared by the Triton-X cleared lysate procedure of Katz et al. (1973) and banded on a CsCl-EtBr buoyant density gradient. Small preps were prepared in the same way, omitting the CsCl gradient and replacing it with a 30 min incubation at 37°C in 100 μg/ml proteinase K, followed by two extractions with an equal volume of phenol, and ethanol precipitation. Transformation was carried out as described by Cohen et al. (1972).

As disclosed above, the subject process is useful to stabilize foreign proteins in the E. coli cell. Proteins which are susceptible to the proteases within the E. coli cell are numerous and well known. Examples are somatostatin and human beta preinterferon. Other genes encoding sequences for the production of useful foreign proteins can be inserted into T4 by use of the techniques disclosed herein with suitable obvious adjustments which are well within the skill of those in the art. As is evident, such an inserted gene would contain the essential control signals (promoter and ribosome initiation site) which are actively recognized in a T4 infected cell, i.e., signals derived from T4 itself. Where it is desired to transfer large DNA inserts into T4, appropriate deletion mutants of T4 will be used. Such deletion mutants of T4 are well known and readily available to persons skilled in the art. Thus upon the obtention of suitable T4 strains with compensatory deletions to accommodate various sizes of DNA gene inserts, a person skilled in the art, with the subject disclosure, is enabled to clone various genes encoding useful foreign proteins into T-even bacteriophage. The final result is the expression of the useful foreign protein without degradation by the host E. coli cell. The subject process is also useful in the screening of DNA banks by well-known procedures and antigenic methods. The stabilization of the produced proteins enhances the efficiency of such methods since lower levels of protein can be detected.

The following cultures have been deposited in the permanent collection of the Northern Regional Research Center (NRRL), U.S. Department of Agriculture, Peoria, Illinois, USA, on 7th July 1983:

E. coli RR1 pBR322(H23r⁺), NRRL B-15475
E. coli 1078, NRRL B-15476
E. coli 1078 (pNC7), NRRL B-15477
E. coli 1078 (pNC7lac2), NRRL B-15478.

Plasmid pBR322 is a well-known and available plasmid. It is maintained in the E. coli host ATCC 37017.

Purified pBR322 DNA, pNC7 DNA and pNC7/lac2 DNA can be obtained from their *E. coli* hosts by well-known procedures, e.g., using the cleared lysate-isopycnic density gradient procedures.

Following are examples which illustrate procedures, including the best mode, for practicing the invention, and products produced thereby. These examples should not be construed as limiting. All percentages are by weight and solvent mixture proportions are by volume unless otherwise noted.

Example 1
Construction and characterization of plasmid pNC7

pBR322(H23r⁺) contains an 873-bp *Hind*III fragment of the *r*l genes of T4 (HH870) inserted into the single *Hind*III site of pBR322. This fragment spans the carboxy terminal coding region of *r*IA, the 11-bp intercistronic region and the amino terminal coding region of the *r*IIB gene. The sequences of pBR322 (Sutcliffe, 1978) and HH870 (Pribnow et al., 1981) are known, enabling restriction enzyme sites and sizes of fragments to be predicted for the chimeric plasmid. The orientation of HH870 within pBR322 was easily determined by a double digestion with the restriction endonucleases *Eco*RI and *Hinc*II. The smallest *Hinc*II fragment of H32r⁺ was found to harbor the *Eco*RI site, revealing the orientation to be counterclockwise from *r*IIA to B in the plasmid. A convenient site for insertion of DNA fragments to be cloned into T4, within HH870, is a *Hinc*II site located 110-bp into the *r*IIB gene, the only *Hinc*II site in HH870. There are two additional *Hinc*II sites in pBR322, one in the ampicillin-resistance gene (at position 3907) and another at position 652. To facilitate insertion of fragments of interest into the *Hinc*II site located within HH870, the site located at position 652, which is also recognized by *Sal*I, was eliminated. After cleaving with *Sal*I, 5' single-strand extensions were filled in with the four deoxynucleotide triphosphates and Klenow fragment of DNA polymerase I, and the resulting ends were ligated back together. During this procedure plasmids were isolated that contained deletions of various sizes. One of these, pNC7, was chosen to be used for the remainder of the experiments.

The plasmid pNC7 is about 3.67 kb in length and confers resistance to ampicillin. Extensive restriction analyses were performed on pNC7, pBR322 and the parental plasmid H23r⁺ to estimate the size and endpoints of the pNC7 deletion. As expected, the *Sal*I site at position 650 of pBR322 and the *Hinc*II site at position 652, were lost. In the counterclockwise direction, all restriction sites remained intact, including the *Hin*fI site at position 631 of pBR322. In the clockwise direction, the *Hha*I site at position 2180 was clearly lost, while the site at 2210 was still intact. Thus the clockwise endpoint of the deletion is at position 2194±13 bp. Size analysis of the new *Alu*I and *Hin*fI restriction fragments generated by the deletion, is consistent with a deletion of about 1546 bp. This suggests that the deletion extends in a single clockwise direction from the *Sal*I site to approximately position 2196 of pBR322.

Example 2
Insertion of the *lac* fragment into plasmid pNC7

A fragment of the control region of the lactose operon of *E. coli* was chosen as the DNA to be cloned, due to its known nucleotide sequence (Maizels, 1973; Dickson et al., 1975; Farabaugh, 1978), ease of availability, and the advantages it offers for selection. A 203-bp fragment that begins 17 codons from the end of the *lac*I gene and extends through the operator/promoter region to the 8th codon of the *lac*Z gene, was removed from the plasmid pOP203-1 by *Eco*RI digestion. This mixture of fragments was then treated with S1 nuclease to remove the 4-bp 5' single-strand extensions, phenol extracted and blunt-end ligated to pNC7 plasmid, which had been partially cleaved with *Hinc*II to yield mostly linear molecules. The ligated mixture was then used to transform *E. coli* 1078 and the transformants were selected on plates containing ampicillin, thereby selecting against plasmids containing fragments inserted into the remaining *Hinc*II site in the pBR322 DNA. Xgal was also included in the plates to allow easy identification of colonies that contained plasmids harboring the *lac* control fragment. Because this plasmid is present in high copy number, the presence of the *lac* operator on the plasmid is sufficient to titrate all the *lac* repressor from the chromosomal operator. The result is constitutive production of β-galactoside, which cleaves the chromogenic substrate, generating bright blue colonies on the plate.

Promising candidates were chosen from the ampicillin-Xgal plates and plasmid DNA was isolated in sufficient quantities to do several restriction enzyme reactions. The plasmid pNC7/lac2 was determined to be approximately 200 bp larger than pNC7 by restriction analysis with *Eco*RI, *Hind*III and *Bgl*I. Digestion with *Alu*I was consistent with this insertion being the *lac* fragment, as the internal *lac Alu*I fragments were present, and the pNC7 fragments with the adjoining *lac* ends were the appropriate sizes. *Hin*fI, which cleaves near both ends of the *lac* DNA, also generated a fragment of the expected size. A *Hha*I restriction site asymmetrically located within the 203-bp *lac* fragment allowed determination of the orientation of the *lac* fragment within the *r*II region of the plasmid. It was found to be oriented such that the operator/promoter region would drive transcription toward the *r*IIB gene, or in a counterclockwise direction from *lac*I to *lac*Z in the plasmid.

Example 3
Transfer of the *lac* fragment into T4

Mattson et al. (1977) reported that recombination can occur *in vivo* between *E. coli* plasmids containing T4 sequences and the T4 genome. Recombination across the *r*II sequences of pNC7/lac2 allows transfer of

4

the *lac* sequences from the plasmid into the phage genome. The *lac* fragment contains translational termination codons in all reading frames (Dickson et al., 1975; Farabaugh, 1978). Since the *Hinc*II restriction site is within the coding region of the *rll*B gene, *lac* insertion would cause the recombinant phage to be *rll*B⁻. It has previously been shown (Berger and Kozinski, 1969; Ebisuzaki and Campbell, 1969; Karam, 1969) that mutations in gene 30 (DNA ligase) of T4, normally a lethal phenotype, can be suppressed on certain strains of *E. coli* by a compensatory mutation in *rll*. Although the mechanism remains undefined, this offers an easy means of selecting T4 phage that have acquired the *lac* insertion. The transfer and selection was accomplished in one step by infecting an amber suppressor *E. coli* strain containing the plasmid (1078 [pNC7*lac*2]) with C104B, a T4 strain that is *rll*⁺ $30_{am}^-$. Progeny of the infection were selected on *E. coli* strain BB, which does not suppress amber mutations, but does allow suppression of gene 30 mutations by mutations in *rll* (Karam and Barker, 1971). Frequencies of rescue of the gene 30 mutation (the fraction of total phage after the infection that could grow on the non-amber suppressing strain) were in the range of $1 \times 10^{-2}$ to $4 \times 10^{-3}$ (Table I), which was a 10- to 18-fold increase over the frequency for the progeny produced during an infection of a strain bearing the plasmid-containing wild-type *rll* DNA.

TABLE I
Efficiency of plating fo C104B on plasmid-containing *E. coli* strains

*E. coli* 1078 carrying the plasmid indicated was infected with C104B at a multiplicity of 1.0. After 5 min the culture was diluted in H broth (Steinberg and Edgar, 1962) and incubated at 30°C. The culture was lysed after 2 h and progeny of the infection plated. The efficiency of plating is the fraction of total progeny (plaques on *E. coli* $B_{40}$sul⁺) that could plate on the non-amber suppressing strain *E. coli* BB.

| | Efficiency of plating | |
|---|---|---|
| Experiment | pNC7($\times 10^{-4}$) | pNC7*lac*2 |
| 1 | 2.4 | $3.5 \times 10^{-3}$ |
| 2 | 4.9 | $9.0 \times 10^{-3}$ |
| 3 | 7.0 | $1.1 \times 10^{-2}$ |
| 4 | 4.0 | $4.0 \times 10^{-3}$ |

Phage containing the *lac* insert were quite stable. During the preparation of a phage stock from a single isolated plaque, revertants, identified by the ability to grow on CR63 (λ), occurred at a frequency of about $1 \times 10^{-9}$. Analysis of the proteins produced by four of these revertants, by gel electrophoresis, showed *rll*B proteins of molecular weight indistinguishable from wild type. Therefore, revertants arise by an almost exact, in-frame deletion of the *lac* insert.

Example 4
The *lac* fragment is part of the T4 genome

Evidence that the mutation mapping at the *rll*B *Hinc*II site is, in fact, due to insertion of the 203-bp *lac* DNA was provided by restriction analysis. T4 DNA in its native form is completely substituted with glcHMC. HMC DNA can be obtained by infecting an *E. coli* strain that lacks UDP-glucose pyrophosphorylase and the host nuclease which degrades HMC DNA (Revel and Georgopoulos, 1969). Unmodified cytosine-containing T4 DNA can also be obtained, but this requires mutations in additional phage genes (Snyder et al., 1976). Two restriction endonucleases have recently been found to function on native T4 DNA: *Eco*RV and *Taq*I. Two additional enzymes can function on HMC T4 DNA: *Eco*RI (Kaplan and Nierlich, 1975) and *Xba*I. To obtain complete digestion of HMC T4 DNA with *Eco*RI requires large amounts of enzyme and long digestion times.

Work on bacteriophage SP01 DNA, which also contains a modified base (thymine is completely replaced by HMU), indicated that the same digestion pattern generated under *Eco*RI conditions, can be generated under *Eco*RI* conditions in a shorter length of time with much less enzyme (Cregg and Stewart, 1978). Digestion of HMC T4 DNA under *Eco*RI* conditions showed this same increase in reaction rate without loss of specificity.

HMC T4 DNA was prepared from wild type and *lac* insert phage. Digestion of these DNAs under *Eco*RI* conditions reveals a shift in the position of a band at about 5.4 kb in wild-type DNA, to 5.6 kb in the *lac*-containing DNA. It was fortuitous that the *Eco*RI* bands containing the *rll* genes migrates to a position that is relatively free from other bands that might mask the change in $M_r$-value. Previously the best estimate for the size of the *Eco*RI fragment containing the *rll* genes was about 4.4 kb (Selzer et al., 1978), which was determined from the size of an *Eco*RI fragment of cytosine-containing T4 DNA. To obtain unmodified DNA a deletion was introduced in the region adjacent to the rII genes; therefore, the size of the wild-type fragment was based upon the estimated size of the deletion used.

HMC DNA from wild-type and lac insertion phage was also digested with restriction endonuclease TaqI. Again a shift in the size of a band is apparent in the lac-containing DNA. Material from the wild-type digest, migrating as part of a band at 1520 bp, migrates as part of a band at 1730 bp in the digest from T4 rIIBlac2. These sizes are expected to be slight overestimates, since the $M_r$ standards from which they were computed contained cytosine rather than HMC. The sizes are consistent with those expected. The lac203 DNA was inserted into the unique HincII cleavage site (between bp 543 and 544) of HH870. The latter has only one TaqI recognition sequence, about 20 bp from the right end (Pribnow et al., 1981). The HindIII fragment immediately to the left of HH870 has about 400 bp (Selzer et al., 1978) and is not cut by TaqI. Thus, the TaqI fragment expected to be altered by insertion should contain at least 1200 bp. Since the lac DNA used has no cleavage site for TaqI (Maizels, 1973; Dickson et al., 1975; Farabaugh, 1978), we would expect the insertion to result in an increase of 203 bp. To prove that these two bands do indeed contain the rII and lac DNA, separate Southern (1975) hybridizations were done to duplicate lanes of this gel with the plasmid pNC7 as a probe for the rII DNA and M13mp2 RF as a probe for the presence of the lac fragment. An autoradiogram of the hybridization with the pNC7 probe shows that these two bands are the only ones containing DNA complementary to HH870. An autoradiogram of the hybridization to the same gel with M13mp2 RF as a probe shows that the lac fragment is indeed physically inserted into the rII genes.

References

Backman, K. Ptashne, M. and Gilbert, W.: Construction of plasmids carrying the cl gene of bacteriophage λ. Proc. Natl. Acad. Sci. USA 73 (1976) 487—560.

Barnett, L., Brenner, S., Crick, F. H. C., Shulman, R. G. and Watts-Tobin, R. J.: Phase shift and other mutants in the first part of the rIIB cistron of bacteriophage T4. Phil. Trans. Royal Soc. London, Series B 252 (1976) 487—560.

Benzer, S.: On the topography of the genetic fine structure. Proc. Natl. Acad. Sci. USA 45 (1959) 1607—1620.

Benzer, S. and Champe, S. P.: A change from nonsense to sense in the genetic code. Proc. Natl. Acad. Sci. USA 48 (1962) 1114—1121.

Berger, H. and Kozinski, A. W.: Suppression of T4D ligase mutations by rIIA and rIIB mutations. Proc. Natl. Acad. Sci. USA 64 (1969) 897—904.

Casna, N. J. and Shub, D. A.: Bacteriophage T4 as a generalized DNA-cloning vehicle. Gene 18 (1982) 297—307.

Cohen, S. N., Chang, A. C. Y. and Hsu, L.: Nonchromosomal antibiotic resistance in bacteria: genetic transformation of Escherichia coli by R-factor DNA. Proc. Natl. Acad. Sci. USA 69 (1972) 2110—2114.

Cregg, J. M. and Stewart, C. R.: EcoRI cleavage of DNA from Bacillus subtilis phage SP01. Virology 85 (1978) 601—605.

Dickson, R. C. , Abelson, J., Barnes, W. M. and Reznikoff, W. S.: Genetic regulation: the lac control region. Science 187 (1975) 27—35.

Ebisuzaki, K. and Campbell, L.: On the role of ligase in genetic recombination in bacteriophage T4. Virology 38 (1969) 701—703.

Farabaugh, P. J.: Sequence of the lacI gene. Nature 274 (1978) 765—769.

Kaplan, D. A. and Nierlich, D. P.: Cleavage of nonglucosylated bacteriophage T4 deoxyribonucleic acid by restriction endonuclease EcoRI. J. Biol. Chem. 250 (1975) 2395—2397.

Karam, J. D.: DNA replication by phage T4rII mutants without polynucleotide ligase (gene 30). Biochem. Biophys. Res. Commun. 37 (1969) 416—422.

Karam, J. D. and Barker, B.: Properties of bacteriophage T4 mutants defective in gene 30 (deoxyribonucleic acid ligase) and the rII gene. J. Virol. 7 (1971) 260—266.

Katz, L., Kingsbury, D. T. and Helinski, D. R.: Stimulation by cyclic adenosine monophosphate of plasmid deoxyribonucleic acid replication and catabolite repression of the plasmid deoxyribonucleic acid-protein complex. J. Bacteriol. 114 (1973) 577—591.

Maizels, N. M.: The nucleotide sequence of the lactose messenger ribonucleic acid transcribed from the UV5 promoter mutant of Escherichia coli. Proc. Natl. Acad. Sci. USA 70 (1973) 3585—3589.

Mattson, T., Van Houwe, G., Bolle, A., Selzer, G. and Epstein, R.: Genetic identification of cloned fragments of bacteriophage T4 DNA and complementation by some clones containing early T4 genes. Mol. Gen. Genet. 154 (1977) 319—326.

Polisky, B., Greene, P., Garfin, D. E., McCarthy, B. J., Goodman, H. M. and Boyer, H. W.: Specificity of substrate recognition by the EcoRI restriction endonuclease. Proc. Natl. Acad. Sci. USA 72 (1975) 3310—3314.

Pribnow, D., Sigurdson, D. C., Gold, L., Singer, B. S., Napoli, C., Brosius, J., Dull, T. J. and Noller, H. F.: rII cistrons of bacteriophage T4. DNA sequence around the intercistronic divide and positions of genetic landmarks. J. Mol. Biol. 148 (1981) 337—376.

Revel, H. and Georgopoulos, C. P.: Restriction of nonglucosylated T-even bacteriophages by prophage P1. Virology 39 (1969) 1—17.

Selzer, G., Bolle, A., Krisch, H. and Epstein, R.: Construction and properties of recombinant plasmids

containing the *r*II genes of bacteriophage T4. Molec. Gen. Genet. 159 (1978) 301—309.

Shub, D. A. and Casna, N. J.: Bacteriophage T4 as a generalized DNA cloning vector. Amer. Soc. Microbiol. Abstracts of the Annual Meeting (1981) H3, 114.

Simon, L.D., et al. Stabilization of proteins by a bacteriophage T4 gene cloned in *Escherichia coli.* Proc. Natl. Acad. Sci. USA 80 (1983) 2059—2062.

Snyder, L., Gold, L. and Kutter, E.: A gene of bacteriophage T4 whose product prevents true late transcription on cytosine-containing T4 DNA. Proc. Natl. Acad. Sci. USA 73 (1976) 3098—3102.

Southern, E. M.: Detection of specific sequences among DNA fragments separated by gel electrophoresis. J. Mol. Biol. 98 (1975) 503—517.

Steinberg, C. M. and Edgar, R. S.: A critical test of a current theory of genetic recombination in bacteriophage. Genetics 47 (1962) 187—208.

Sutcliffe, G.: pBR322 restriction map derived from the DNA sequence: accurate DNA size markers up to 4361 nucleotide pairs long. Nucl. Acids Res. 5 (1978) 2721—2728.

## Claims

1. A process for stabilising proteins which can be expressed by *E. coli* via a cloned gene and which may be susceptible to proteases produced within the *E. coli* cell, the process comprising:

(a) constructing a chimeric plasmid comprising a DNA fragment from an *E. coli* plasmid and a T-even bacteriophage which is stably propagated in an *E. coli* bacterium;

(b) inserting foreign DNA, *in vitro*, into the DNA fragment from a T-even bacteriophage of the chimeric plasmid, to give a doubly chimeric plasmid;

(c) recombining, *in vivo*, a T-even bacteriophage and the doubly chimeric plasmid, resulting in the insertion of the foreign DNA into the bacteriophage; and

(d) infecting an *E. coli* bacterium with T-even bacteriophage harbouring the foreign DNA.

2. A process according to claim 1, wherein the DNA comprises the *r*II genes of bacteriophage T4.

3. A process according to claim 1 or claim 2, wherein the T-even bacteriophage in step (c) is bacteriophage T4.

4. A process according to any preceding claim, wherein the *E. coli* plasmid is pBR322(H23r$^+$).

5. A process according to any preceding claim, wherein the foreign DNA insert is a eukaryotic gene encoding a polypeptide or protein.

6. A process according to any preceding claim, wherein the *E. coli* bacterium is *E. coli* 1078 (C600 r$^-$m$^+$ *sup*E44 *thr*$^-$ *leu*$^-$ *thi*$^-$) or *E. coli* (C600 *sup*E44).

7. Plasmid pNC7, characterised in that it (1) contains a portion of the genome of plasmid pBR322)H23r$^+$) including an 873-bp *Hind*III fragment of the *r*II genes of bacteriophage T4 (HH870) inserted into the single *Hind*III site of pBR322; (2) is about 3.67 kb in length; and (3) confers resistance to ampicillin.

8. Plasmid pNC71*lac*2, characterised in that it (1) contains a portion of the genome of plasmid pBR322(H23r$^+$) including an 873-bp *Hind*III fragment of the *r*II genes of bacteriophage T4 (HH870) inserted into the single *Hind*III site of pBR322; (2) confers resistance to ampicillin; and (3) makes host cells carrying a functional β-galactosidase gene, a constitutive producer of β-galactoside.

## Patentansprüche

1. Verfahren zum Stabilisieren von Proteinen, die durch *E. coli* über ein geklontes Gen exprimiert werden können und gegenüber Proteasen, die in der *E. coli*-Zelle gebildet werden, empfindlich sein können, das umfaßt:

(a) Konstruktion eines chimären Plasmids, das ein DNA-Fragment aus einem *E. coli*-Plasmid und einem T-geraden Bakteriophagen umfaßt, das stabil in einem *E. coli*-Bakterium vermehrt wird;

(b) in vitro Insertion einer Fremd-DNA in das DNA-Fragment des T-geraden Bakteriophagen des chimären Plasmids zur Bildung eines doppelt chimären Plasmids;

(c) in vivo Rekombination eines T-geraden Bakteriophagen und des doppelt chimären Plasmids, die zur Insertion der Fremd-DNA in den Bakteriophagen führt; und

(d) Infektion eines *E. coli*-Bakteriums mit dem die Fremd-DNA tragenden T-geraden Bakteriophagen.

2. Verfahren gemäß Anspruch 1, worin die DNA die *r*II-Gene des Bakteriophagen T4 umfaßt.

3. Verfahren gemäß Anspruch 1 oder 2, worin der T-gerade Bakteriophage in Stufe (c) Bakteriophage T4 ist.

4. Verfahren gemäß irgendeinem vorhergehenden Anspruch, worin das *E. coli*-Plasmid pBR322(H23r$^+$) ist.

5. Verfahren gemäß irgendeinem vorhergehenden Anspruch, worin das Fremd-DNA-Insert ein eukariontisches, ein Polypeptid oder Protein kodierendes Gen ist.

6. Verfahren gemäß irgendeinem vorhergehenden Anspruch, worin das *E. coli*-Bakterium *E. coli* 1078 (C600 r$^-$m$^+$ *sup*E44 *thr*$^-$ *leu*$^-$ *thi*$^-$) oder *E. coli* (C600 *sup*E44) ist.

7. Plasmid pNC7, dadurch gekennzeichnet, daß es (1) einen Teil des genoms von Plasmid pBR322(H23r$^+$) einschließlich eines 873bp *Hind*III-Fragmentes der *r*II-Gene des Bakteriophagen T4 (HH870), insertiert in die einzige *Hind*III-Stelle von pBR322, enthält; (2) eine Länge von etwa 3,67 kb besitzt und (3) Ampicillinresistenz verleiht.

8. Plasmid pNC71*lac*2, dadurch gekennzeichnet, daß es (1) einen Teil des Genoms von Plasmid pBR322(H23r⁺) einschließlich eines 873bp *Hind*III-Fragmentes der *r*II-Gene des Bakteriophagen T4 (HH870), insertiert in die einzige *Hind*III-Stelle von pBR322, enthält; (2) Ampicillinresistenz verleiht; und (3) Wirtszellen, die ein funktionelles β-Galactosidase-Gen tragen, zu einem konstitutiven Produzenten von β-Galactosid macht.

**Revendications**

1. Un procédé pour stabiliser les protéines qui peuvent être exprimées par le *E. coli* via un gène cloné et qui peuvent être sensibles aux protéases produites à l'intérieure de la cellule de *E. coli*, le procédé comprenant:

(a) la construction d'un plasmide chimérique comprenant un fragment d'ADN provenant d'un plasmide de *E. coli* et un bactériophage T-pair qui est propagé de façon stable dans une bactérie *E. coli*;

(b) l'insertion de l'ADN étranger, *in vitro*, dans le fragment d'ADN provenant d'un bactériophage T-pair du plasmide chimérique pour donner un plasmide chimérique doublé;

(c) la recombinaison, *in vitro*, d'un bactériophage T-pair et du plasmide chimérique doublé, résultant en une insertion de l'ADN étranger dans le bactériophage; et

(d) l'infection d'une bactérie *E. coli* avec le bactériophage T-pair contenant l'ADN étranger.

2. Un procédé selon la revendication 1, selon lequel l'ADN comprend les gènes *r*II du bactériophage T4.

3. Un procédé selon la revendication 1 ou 2, selon lequel le bactériophage T-pair dans l'étape (a) est le bactériophage T4.

4. Un procédé selon l'une quelconque des revendications précédentes, selon lequel le plasmide de *E. coli* est le pBR322(H23r⁺).

5. Un procédé selon l'une quelconque des revendications précédentes, selon lequel la séquence d'insertion d'ADN étranger est un gène eucaryote codant pour un polypeptide ou une protéine.

6. Un procédé selon l'une quelconque des revendications précédentes, selon lequel la bactérie *E. coli* est le *E. coli* 1078 (C600 r⁻m⁺ *sup*E44 *thr⁻ Leu⁻ thi⁻*) ou le *E. coli* (C600 *sup*E44).

7. Plasmide pNC7, caractérisé en ce qu'il (1) contient une portion du génome du plasmide pBR322(H23r⁺) incluant un fragment de 873-bp *Hind*III des gènes *r*II du bactériophage T4 (HH870) inséré dans le seul site *Hind*III du pBR322; (2) a une longueur d'environ 3,67 kb; et (3) confère la résistance à l'ampicilline.

8. Plasmide pNC71*lac*2, caractérisé en ce qu'il (1) contient une portion du génome du plasmide pBR322(H23r⁺) incluant un fragment de 873-bp *Hind*III des gènes *r*II du bactériophage T4 (HH870) inséré dans le seuil site *Hind*III du pBR322; (2) confère la résistance à l'ampicilline; et (3) rend les cellules hôtes, porteuses d'un gène fonctionnel β-galactosidase, productrices constitutives du β-galactoside.